# EUROPÄISCHE PATENTSCHRIFT

(11) **EP 0 900 055 B1**
(45) Veröffentlichungstag und Bekanntmachung des Hinweises auf die Patenterteilung: **17.09.2003**
(21) Anmeldenummer: 98904013.4
(22) Anmeldetag: 07.01.1998
(51) Int. Cl.: A61B 19/00, B25F 5/00

(54) **INSTRUMENT ZUR KOMPENSATION DES HANDZITTERNS BEI DER MANIPULATION FEINER STRUKTUREN**
INSTRUMENT FOR COMPENSATING HAND TREMBLING IN THE MANIPULATION OF FINE STRUCTURES
INSTRUMENT POUR COMPENSER LE TREMBLEMENT DE LA MAIN LORS DE LA MANIPULATION DE FINES STRUCTURES

(30) Priorität: 08.01.1997 DE 19700402
(43) Veröffentlichungstag der Anmeldung: 10.03.1999
(73) Patentinhaber: Peer, Ferdinand, Dr., 80801 München (DE)
(72) Erfinder: Peer, Ferdinand, Dr., 80801 München (DE)
(74) Vertreter: Goddar, Heinz J., Dr.
(86) Internationale Anmeldenummer: DE9800043
(87) Internationale Veröffentlichungsnummer: WO98030165

(56) Entgegenhaltungen:
- EP-A- 0 425 352
- EP-A- 0 715 092
- US-A- 4 919 152
- US-A- 4 944 741
- US-A- 5 209 326
- US-A- 5 312 434
- US-A- 5 571 137

## Beschreibung

Das manuelle Arbeiten an feinen Strukturen wird durch die als Zittern oder Tremor bezeichnete unwillkürlich auftretende, weitgehend rhythmisch aufeinanderfolgende Kontraktion antagonistisch wirkender Muskeln erheblich erschwert. Dies macht sich insbesondere in der Mikrochirurgie bei Nähten an feinen Nerven oder Gefäßen bemerkbar, verlängert die Operationsdauer und verringert die Qualität des Ergebnisses.

Aus US-PS 4 919 152 und US-PS 4 944 741 sind chirurgische Instrumente, insbesondere für endoskopische Verfahren, bekannt, die auf einer Seite Greifelemente, beispielsweise in Form zweier Klemmbacken, besitzen, welche über einen langen Stiel von einem handgehaltenen Abschnitt auf der anderen Seite mit Hilfe von Federn bedient werden können. Diese Instrumente besitzen jedoch den Nachteil, daß durch Zittern hervorgerufene unerwünschte Bewegungen an die Arbeitsspitze weitergegeben werden.

Um dennoch filigrane Tätigkeiten durchführen zu können, verwendet man zum Beispiel Auflagen für die Hände oder Mikromanipulatoren, welche gröbere Bewegungen in feine Auslenkungen umsetzen.

Es ist die Aufgabe der Erfindung, ein hangehaltenes Instrument zur manuellen Manipulation feiner Strukturen zu schaffen, bei dem sich unerwünschte Bewegungen, zum Beispiel Tremor, nicht oder zumindest weniger stark auf die Manipulation auswirken.

Der Erfindung liegt der Gedanke zugrunde, bei handgehaltenen Instrumenten die unerwünschten Bewegungen zu evaluieren und durch Aktoren die Arbeitsspitze des Instruments - bei chirurgischen Anwendungen wäre dies beispielsweise Nadelhalter - zur Kompensation so auszulenken, daß sich das Handzittern an der Spitze nicht bemerkbar macht.

Da sich der physiologische Tremor in einem Bereich zwischen etwa 5 und 15 Schlägen/Sekunde abspielt, kann er von langsamer ablaufenden Willkürbewegungen, beispielsweise durch eine frequenzselektive Filterung, unterschieden werden.

### Bewegungserfassung

Erfindungsgemäß können eine oder mehrere Einrichtungen zum Erfassen von Bewegungen des Benutzers, insbesondere zum Erfassen von durch den Benutzer verursachten Bewegungen des Instruments vorgesehen sein, beispielsweise aktive oder passive Meßwertaufnehmer, mit Hilfe derer sich unerwünschte Bewegungen des Benutzers bzw. durch den Benutzer verursachte unerwünschte Bewegungen des Instruments erfassen lassen. Die Ausgangssignale dieser Einrichtungen werden, ggf. nach entsprechender Auswertung und Verarbeitung, zur Ansteuerung von Aktoren des Instruments zum Ausgleich der unerwünschten Bewegungen herangezogen.

Zur Erfassung der Bewegung des Instruments stehen prinzipiell mehrere, auch untereinander kombinierbare Möglichkeiten zur Auswahl:
1. An einem oder mehreren Abschnitten des Instruments sind Beschleunigungs- und Winkelgeschwindigkeitssensoren angebracht, die ein mit der Bewegung des Instruments korreliertes mechanisches oder elektrisches Signal liefern.
   Für die Realisierung der Sensoren kommen rein mechanische, elektromagnetische, kapazitive, piezoelektrische oder -resistive Funktionsprinzipen in Frage. Die Sensoren müssen die Bewegungen mit ausreichender Empfindlichkeit und Genauigkeit erfassen. Dabei ist der Einsatz von Sensoren denkbar, die ihr Empfindlichkeitsmaximum in dem Frequenzbereich aufweisen, in dem sich die unerwünschten Bewegungen abspielen und damit ein mit dem Tremor korreliertes Ausgangssignal liefern. Die Sensoren können so angeordnet sein, daß sie sowohl Translationsals auch Rotationsbewegungen des Instruments erfassen können.
2. Erfindungsgemäß können eine oder mehrere Einrichtungen zur repetitiven oder kontinuierlichen Erfassung der Position bestimmter Abschnitte des Instruments, insbesondere eines handgehaltenen Abschnitts und/oder eines gegenüber dem handgehaltenen Abschnitt beweglichen Abschnitts des Instruments, in einer, zwei oder drei Dimensionen vorgesehen sein. Über eine repetitive oder kontinuierliche Erfassung von Lage und Orientierung bestimmter Abschnitte des Instruments läßt sich deren Bewegung verfolgen. Die Erfassung kann in einer, zwei oder drei Dimensionen stattfinden. Dies kann drahtlos über ein Sender-Empfänger-System am Instrument und an Bezugspunkten, die sich im fest im Raum oder auf dem bearbeiteten Objekt befinden, realisiert sein.
   Es ist, insbesondere bei der Anwendung in der Mikrochirurgie auch möglich, die Bewegung des sich im Operationsmikroskop darstellenden Abschnitts des Instrumentes zu verfolgen. Das Mikroskopbild wird einer Bilderfassungseinheit zugeführt, welche die Positionen von bestimmten Merkmalen oder optischen Markierungen auf dem Instrument in der Abbildung bestimmt und anhand der zeitlich aufeinanderfolgenden Positionsdaten die Bewegung errechnet. Dazu können auch als "Tracking" bekannte Objektverfolgungsverfahren herangezogen werden.
   Erfaßt man zusätzlich die sich ebenfalls im Operationsmikroskop darstellende Bewegung des bearbeiteten Gewebes, so kann dessen beispielsweise durch Zittern des Patienten verursachte Bewegung mit in den Kompensationsprozeß einbezogen werden.
3. Mit auf der Haut des Instrumentenbedieners angebrachten Oberflächenelektroden können die Aktionspotentiale des darunterliegenden Muskels registriert werden (sog. Elektromyographie). Ist einem Regelsystem -beispielsweise aufgrund der Fähigkeit zur Adaptation- die Bewegung, die der entsprechende Muskel bei Innervation hervorruft, bekannt, kann durch Kombination der Elektrodensignale auf die am Instrument zu erwartende Auslenkung geschlossen werden.
   Bei diesem Verfahren würde dem eigentlichen Einsatz des Instruments eine "Lernphase" vorangehen, in der die Signalverarbeitungseinheit die elektromyographischen Signale mit Ergebnissen anderer Bewegungserfassungsverfahren korreliert und sich dadurch auf individuelle Merkmale des Bedieners und auf die Positionierung der Ableitelektroden einstellt.

### Signalverarbeitung

Soweit die Bewegungserfassung nicht bereits ein Signal liefert, das der auf den Tremor zurückzuführenden Bewegung entspricht und damit zur direkten Ansteuerung der Aktoren geeignet ist, wird das Signal einer Verarbeitung unterzogen.
Im allgemein wird zunächst eine Verstärkung des Sensorsignals notwendig sein. Daran schließt sich eine Analyse an, die anhand von Kriterien wie Frequenz, Amplitude, Geschwindigkeit und Richtung beabsichtigte von unabsichtlichen Bewegungen unterschiedet. Die Kriterien können vorgegeben sein, sich einstellen lassen oder sich selbständig anpassen. Anhand der Daten wird ein Signal gewonnen, mit dem die Aktoren so angesteuert werden, daß diese an der Arbeitsspitze des Instruments Relativbewegungen zur Kompensation der unerwünschten Auslenkungen des handgehaltenen Abschnitts ausführen.

### Aktorik

Als Aktoren kommen sowohl rein mechanische Anordnungen als auch elektrische Stellglieder, die beispielsweise auf elektromagnetischen, kapazitiven oder piezoelektrischen Prinzipien basieren, in Frage. Die Aktoren führen Relativbewegungen zwischen dem handgehaltenen und dem beweglichen Abschnitt des Instruments aus. Sie müssen die gewünschten Bewegungen mit ausreichender Geschwindigkeit, Kraft und Präzision ausführen können. Die Aktoren können so angeordnet sein daß sie sowohl Translations- als auch Rotationsbewegungen bewirken können. Für bestimmte Anforderungen kann es wünschenswert sein, daß die Aktoren auch vorübergehend eine steife Verbindung zwischen dem handgehaltenen und dem beweglichen Abschnitt herstellen können.
Die Arbeitsspitze des Instruments kann sowohl fest montiert als auch auswechselbar ausgelegt sein. Aktionen, wie das Öffnen und Schließen von Greifern oder Pinzetten, die notwendigerweise vom handgehaltenen Abschnitt des Instruments aus erfolgen, können über eine flexible Kraftübertragung, beispielsweise eine biegsamen Schubstange, welche die Aktorik nicht behindert oder über elektrisch betätigte Stellglieder am bewegten Abschnitt ausgelöst werden.

### Zusatzfunktionen

Insbesondere für die Anwendung in der Mikrochirurgie kann es sinnvoll sein, die im Instrument bereits vorhandenen Aktoren auf Knopfdruck so anzusteuern, daß sie die Arbeitsspitze des Instruments in Schwingungen mit Frequenzen im Schall- oder Ultraschallbereich versetzen und - beispielsweise bei der Funktion als Nadelhalter- durch die schnellen Mikrobewegungen das Eindringen einer Nähnadel in Gewebe erleichtern.

### Ausführungsbeispiel

Ein Ausführungsbeispiel der Erfindung ist nachstehend unter Bezugnahme auf die zugehörigen Zeichnungen näher erläutert.

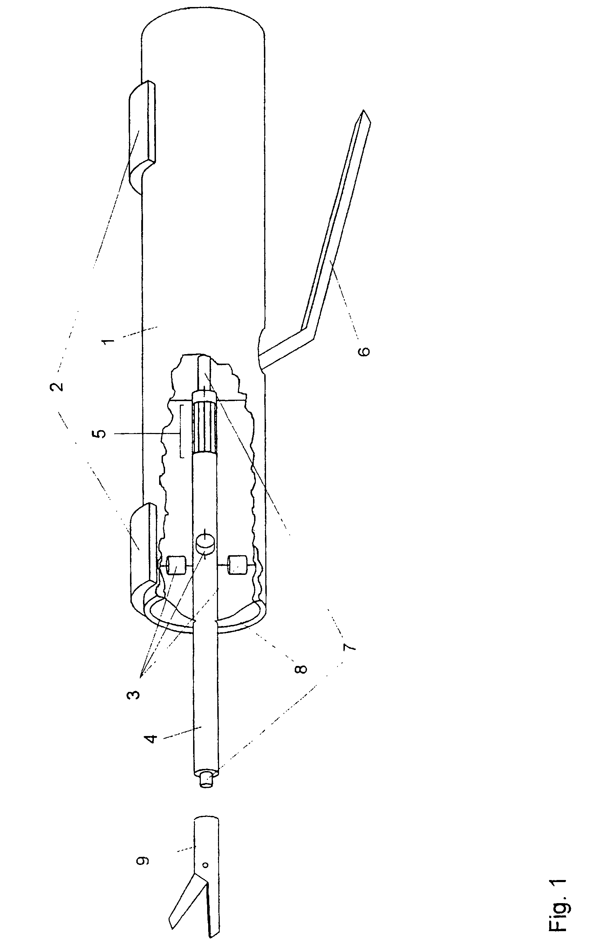

In **Fig. 1** ist mit **(1)** der handgehaltene Abschnitt eines medizinischen Instruments bezeichnet. Auf diesem Abschnitt sitzen Gruppen von Beschleunigungmessern und Winkelgeschwindigkeitssensoren **(2)**. Über eine flexible Verbindung **(5)** ist mit dem handgehaltenen Abschnitt **(1)** ein beweglicher Abschnitt **(4)** verbunden, an dem sich als Arbeitsspitze zum Beispiel ein Nadelhalter befindet. Der bewegliche Abschnitt läßt sich über Anordnung von piezoelektrischen Aktoren **(3)** relativ zum handgehaltenen Abschnitt **(1)** bewegen. Die Aktoren **(3)** sind so angeordnet, daß sie Auslenkungen in alle Richtungen quer zur Hauptachse des Instruments bewirken können.
Im handgehaltenen Abschnitt **(1)** sitzt eine von einer Batterie gespeiste Steuereinheit, welche die von den Sensoren **(2)** gelieferten Daten verstärkt, kontinuierlich analysiert und miteinander verrechnet.

Anhand einstellbarer Kriterien wie Beschleunigung, Frequenz, Richtung und Amplitude der Bewegung unterscheidet die Steuereinheit gewünschte Bewegungen vom Handtremor des Bedieners. Zur Kompensation des Handtremors wird die Anordnung der Aktoren **(3)** in einer Kombination so angesteuert, daß diese nach Richtung, Geschwindigkeit und Amplitude angepaßte Relativbewegungen des beweglichen Teils **(4)** bewirken, und sich die unerwünschten Bewegungen des Handgriffs **(1)** an der Arbeitsspitze des Instrumentenaufnehmers **(4)** nicht bemerkbar machen. Zum Öffnen und Schließen von Nadelhaltern, Pinzetten, oder Scheren dient eine im Bereich der flexiblen Verbindung **(5)** biegsam ausgeführte Schubstange **(7),** die im Inneren des beweglichen Abschnitts **(4)** verläuft. Die Schubstange wird über einen Hebel **(6)** betätigt. Der Abdichtung der Mechanik dient ein flexibler Balg **(8)** an der Stirnseite des handgehaltenen Abschnitts **(1).**

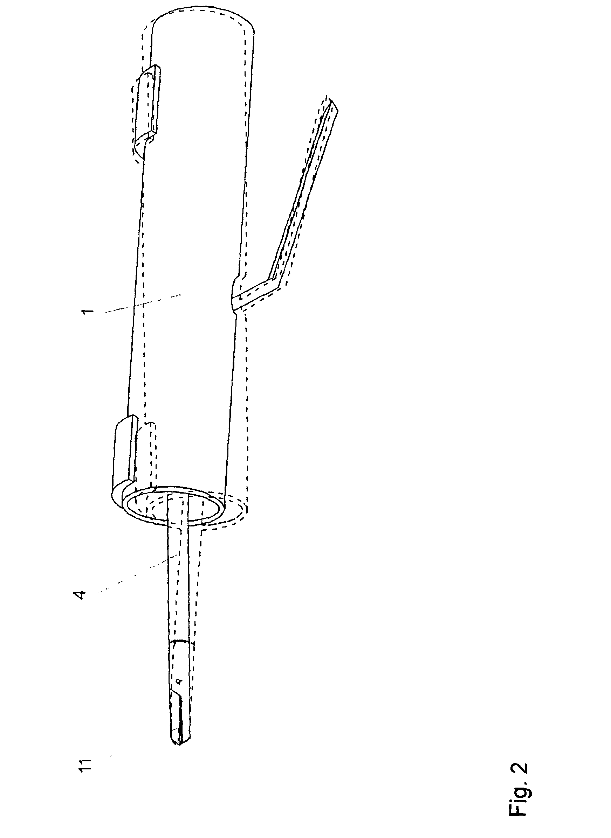

Die Funktion des Instruments ist zusätzlich in **Fig. 2** dargestellt.
Eine schnelle, durch Tremor bedingte, unbeabsichtigte Nickbewegung des handgehalten Abschnitts **(4)** würde zu einer unerwünschten Auslenkung der Arbeitsspitze **(11)** des Instruments führen. Um dies zu verhindern, werden zur Kompensation die Aktoren **(3)** so angesteuert, daß sich der bewegliche Abschnitt **(4)** entgegengesetzt bewegt, und die Arbeitsspitze **(11)** in Ruhe bleibt.

## Patentansprüche

1. Instrument zur manuellen Bearbeitung feiner Strukturen, welches umfaßt:
(a) einem handgehaltenen Abschnitt (1),
(b) einen gegenüber dem handgehaltenen Abschnitt (1) beweglichen Abschnitt (4),
(c) Aktoren (3),
**dadurch gekennzeichnet, daß**
die Aktoren (3) dafür eingerichtet sind, Relativbewegungen zwischen dem handgehaltenen Abschnitt (1) und dem beweglichen (4) Abschnitt zu erzeugen, welche unerwünschten Bewegungen des handgehaltenen Abschnitts (1), die auf unwillkürliche Muskelkontraktionen zurückgehen, entgegengerichtet sind, so daß sich diese unerwünschten Bewegungen nicht oder zumindest weniger stark auf den beweglichen Abschnitt auswirken.

2. Instrument nach Anspruch 1, **dadurch gekennzeichnet, daß** am handgehaltenen Abschnitt **(1)** ein oder mehrere Sensoren **(2)** angebracht sind, welche die Bewegung des handgehaltenen Abschnitts **(1)** erfassen, und deren Ausgangssignal zur Ansteuerung der Aktoren **(3)** herangezogen wird.

3. Instrument nach Anspruch 1 oder 2, **dadurch gekennzeichnet, daß** am beweglichen Abschnitt **(4)** ein oder mehrere Sensoren angebracht sind, welche die Bewegung des beweglichen Abschnitts **(4)** erfassen, und deren Ausgangssignal zur Ansteuerung der Aktoren **(3)** herangezogen wird.

4. Instrument nach einem der Ansprüche 1 bis 3, **dadurch gekennzeichnet, daß** am handgehaltenen Abschnitt **(1)** oder am beweglichen Abschnitt **(4)** oder zwischen dem handgehaltenen **(1)** und dem beweglichen Abschnitt **(4)** ein oder mehrere Sensoren angebracht sind, welche die Relativbewegung zwischen handgehaltenem **(1)** und beweglichem Abschnitts **(4)** erfassen, und deren Ausgangssignal zur Ansteuerung der Aktoren **(3)** herangezogen wird.

5. Instrument nach einem der Ansprüche 2 bis 4, **dadurch gekennzeichnet, daß** zumindest ein Sensor die Beschleunigung erfaßt.

6. Instrument nach einem der Ansprüche 1 bis 5, **dadurch gekennzeichnet, daß** ein oder mehrere Sensoren zur repetitiven oder kontinuierlichen Positionserfassung des handgehaltenen Abschnitts **(1)** in einer, zwei oder drei Dimensionen vorgesehen sind.

7. Instrument nach einem der Ansprüche 1 bis 6, **dadurch gekennzeichnet, daß** ein oder mehrere Sensoren zur repetitiven oder kontinuierlichen Positionserfassung des beweglichen Abschnitts **(4)** in einer, zwei oder drei Dimensionen vorgesehen sind.

8. Instrument nach Anspruch 6 oder 7, **dadurch gekennzeichnet daß** die Positionserfassung des Instruments optisch über den Strahlengang eines Operationsmikroskops erfolgt.

9. Instrument nach einem der Ansprüche 1 bis 8, **dadurch gekennzeichnet, daß** eine Steuer- oder Regeleinheit die Aktoren aufgrund der Daten der Sensoren zur Positionserfassung und/oder der Bewegungserfassung ansteuert.

10. Instrument nach einem der Ansprüche 1 bis 9, **dadurch gekennzeichnet, daß** eine Steuer- oder Regeleinheit die Aktoren aufgrund einer repetitiven oder kontinuierlichen Positionserfassung ansteuert.

11. Instrument nach einem der Ansprüche 1 bis 10, **dadurch gekennzeichnet daß** elektromyographisch erfaßte Potentiale zur Ansteuerung der Aktoren herangezogen werden.

12. Instrument nach einem der Ansprüche 1 bis 11, **dadurch gekennzeichnet, daß** die Kompensationsbewegungen beim Auftreten von Bewegungen des handgehaltenen oder des beweglichen Abschnitts in einem vorbestimmten oder einstellbaren oder sich selbständig anpassenden Frequenzbereich stattfinden.

13. Instrument nach einem der Ansprüche 1 bis 12, **dadurch gekennzeichnet, daß** die Kompensationsbewegungen beim Auftreten von Bewegungen des handgehaltenen oder des beweglichen Abschnitts in einem vorbestimmten oder einstellbaren oder sich selbständig anpassenden Amplitudenbereich stattfinden.

14. Instrument nach einem der Ansprüche 1 bis 13, **dadurch gekennzeichnet, daß** die Aktoren (3) so angesteuert werden können, daß sich eine steife Verbindung zwischen dem handgehaltenen Abschnitt **(1)** und dem beweglichen Abschnitt **(4)** ergibt.

15. Instrument nach einem der Ansprüche 1 bis 14, **dadurch gekennzeichnet, daß** die Aktoren **(3)** so angesteuert werden können, daß sie Schwingungen am beweglichen Abschnitt **(4)** erzeugen können.

## Claims

1. Instrument for manually working on fine structures, comprising:
(a) a handheld section (1),
(b) a section (4) which is movable in relation to the handheld section (1),
(c) actuators (3),
**characterised in that**
the actuators (3) are designed to effect relative movements between the handheld section (1) and the movable section (4), which relative movements are opposite to unwanted movements of the handheld section caused by involuntary muscle contractions so that these unwanted movements have no or at least less effect on the movable section.

2. Instrument according to claim 1, **characterized in that** one or more sensors (2) are arranged on the handheld section (1), said sensors detecting the movement of the handheld section (1), and their output signal being used to control the actuators (3).

3. Instrument according to claim 1 or 2, **characterized in that** one or more sensors are arranged on the movable section (4), said sensors detecting the movement of the movable section (4), and their output signal being used to control the actuators (3).

4. Instrument according to one of claims 1 to 3, **characterised in that** one or more sensors are arranged on the handheld section (1) or on the movable section (4) or between the handheld section (1) and the movable section (4), said sensors detecting the relative movement between the handheld section (1) and the movable section (4), and their output signal being used to control the actuators (3).

5. Instrument according to one of claims 2 to 4, **characterised in that** at least one sensor detects the acceleration.

6. Instrument according to one of claims 1 to 5, **characterised in that** one or more sensors are provided for repetitive or continuous detection of the position of the handheld section (1) in one, two or three dimensions.

7. Instrument according to one of claims 1 to 6, **characterised in that** one or more sensors are provided for repetitive or continuous detection of the position of the movable section (4) in one, two or three dimensions.

8. Instrument according to claim 6 or 7, **characterised in that** position detecting of the instrument is done optically via the ray path of an operating microscope.

9. Instrument according to one of claims 1 to 8, **characterised in that** a control or regulating unit controls the actuators on the basis of the data from the sensors for position detection and/or movement detection.

10. Instrument according to one of claims 1 to 9, **characterised in that** a control or regulating unit controls the actuators on the basis of repetitive or continuous position detection.

11. Instrument according to one of claims 1 to 10, **characterised in that** electromyographically detected potentials are used in controlling the actuators.

12. Instrument according to one of claims 1 to 11, **characterised in that** when movements of the handheld section or movable section occur, the compensation movements take place in a predetermined or adjustable or automatically adapting frequency range.

13. Instrument according to one of claims 1 to 12, **characterised in that** when movements of the handheld section or movable section occur, the compensation movements take place in a predetermined or adjustable or automatically adapting amplitude range.

14. Instrument according to one of claims 1 to 13, **characterised in that** the actuators (3) are capable of being controlled in such a way that a rigid connection is obtained between the handheld section (1) and the movable section (4).

15. Instrument according to one of claims 1 to 14, **characterised in that** the actuators (3) are capable of being controlled in such a way that the actuators can generate oscillations at the movable section (4).

## Revendications

1. Instrument pour le travail manuel de fines structures comprenant :
a) une partie tenue à la main (1),
b) une partie mobile (4) par rapport à la partie tenue à la main (1),
c) des acteurs (3),
**caractérisé en ce que** les acteurs (3) sont ajustés pour générer des mouvements relatifs entre la partie tenue à la main (1) et la partie mobile (4), lesquels sont opposés à des mouvements non souhaités de la partie tenue à la main (1) reposant sur des contractions musculaires involontaires, de sorte que ces mouvements non souhaités n'ont pas d'effet, ou au moins un effet moins important, sur la partie mobile.

2. Instrument selon la revendication 1, **caractérisé en ce qu'**un ou plusieurs capteurs (2) sont fixés sur la partie tenue à la main (1), lesquels saisissent le mouvement de la partie tenue à la main (1), et dont le signal de sortie est pris en compte pour le déclenchement des acteurs (3).

3. Instrument selon la revendication 1 ou 2, **caractérisé en ce qu'**un ou plusieurs capteurs (2) sont fixés sur la partie mobile (4), lesquels saisissent le mouvement de la partie mobile (4), et dont le signal de sortie est pris en compte pour le déclenchement des acteurs (3).

4. Instrument selon l'une quelconque des revendications 1 à 3, **caractérisé en ce que** sur la partie tenue à la main (1) ou sur la partie mobile (4) ou entre la partie tenue à la main (1) et la partie mobile (4), un ou plusieurs capteurs sont fixés, lesquels saisissent le mouvement relatif entre la partie tenue à la main (1) et la partie mobile (4), et dont le signal de sortie est pris en compte pour le déclenchement des acteurs (3).

5. Instrument selon l'une quelconque des revendications 2 à 4, **caractérisé en ce qu'**au moins un capteur saisit l'accélération.

6. Instrument selon l'une quelconque des revendications 1 à 5, **caractérisé en ce qu'**un ou plusieurs capteurs pour la saisie répétitive ou continue de la position de la partie tenue à la main (1) sont prévus en une, deux, ou trois dimensions.

7. Instrument selon l'une quelconque des revendications 1 à 6, **caractérisé en ce qu'**un ou plusieurs capteurs pour la saisie répétitive ou continue de la position de la partie mobile (4) sont prévus en une, deux, ou trois dimensions.

8. Instrument selon la revendication 6 ou 7, **caractérisé en ce que** la saisie de la position s'effectue de manière optique via la trajectoire des rayons d'un microscope de salle d'opération.

9. Instrument selon l'une quelconque des revendications 1 à 8, **caractérisé en ce qu'**une unité de commande ou de réglage déclenche les acteurs en raison des données des capteurs pour la saisie de la position et/ou la saisie du mouvement.

10. Instrument selon l'une quelconque des revendications 1 à 9, **caractérisé en ce qu'**une unité de commande ou de réglage déclenche les acteurs en raison d'une saisie répétitive ou continue de la position.

11. Instrument selon l'une quelconque des revendications 1 à 10, **caractérisé en ce que** les potentiels électromyographiques saisis sont pris en compte pour le déclenchement des acteurs.

12. Instrument selon l'une quelconque des revendications 1 à 11, **caractérisé en ce que** les mouvements de compensation ont lieu lors de l'apparition de mouvements de la partie tenue à la main ou de la partie mobile dans une gamme de fréquences prédéfinie ou réglable ou s'adaptant de manière autonome.

13. Instrument selon l'une quelconque des revendications 1 à 12, **caractérisé en ce que** les mouvements de compensation ont lieu lors de l'apparition de mouvements de la partie tenue à la main ou de la partie mobile dans une gamme d'amplitude prédéfinie ou réglable ou s'adaptant de manière autonome.

14. Instrument selon l'une quelconque des revendications 1 à 13, **caractérisé en ce que** les acteurs (3) peuvent être déclenchés de manière à ce qu'une liaison rigide est obtenue entre la partie tenue à la main (1) et la partie mobile (4).

15. Instrument selon l'une quelconque des revendications 1 à 14, **caractérisé en ce que** les acteurs (3) peuvent être déclenchés de manière à ce qu'ils génèrent des oscillations sur la partie mobile (4).
